(19) Europäisches Patentamt
European Patent Office
Office européen des brevets

(11) **EP 3 441 106 B1**

(12) **EUROPEAN PATENT SPECIFICATION**

(45) Date of publication and mention
of the grant of the patent:
**20.01.2021 Bulletin 2021/03**

(51) Int Cl.:
*A61M 37/00* (2006.01)   *A61L 27/20* (2006.01)
*A61L 27/58* (2006.01)   *A61L 27/54* (2006.01)
*A61F 13/02* (2006.01)   *A61K 9/00* (2006.01)
*A61K 9/70* (2006.01)   *A61K 31/455* (2006.01)

(21) Application number: **17779345.2**

(22) Date of filing: **05.04.2017**

(86) International application number:
**PCT/KR2017/003724**

(87) International publication number:
**WO 2017/176045 (12.10.2017 Gazette 2017/41)**

(54) **MICRONEEDLE STRUCTURE FOR EFFICIENT SKIN PERFORATION**

MIKRONADELSTRUKTUR ZUR EFFIZIENTEN HAUTPERFORATION

STRUCTURE DE MICRO-AIGUILLE POUR UNE PERFORATION ÉFFICACE DE LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.04.2016 KR 20160041577**

(43) Date of publication of application:
**13.02.2019 Bulletin 2019/07**

(73) Proprietor: **LG Household & Health Care Ltd.
Seoul 03184 (KR)**

(72) Inventors:
• **SHIM, Woo-Sun
Daejeon 34114 (KR)**
• **HWANG, Young-Min
Daejeon 34114 (KR)**
• **KANG, Nae-Gyu
Daejeon 34114 (KR)**

(74) Representative: **Goddar, Heinz J.
Boehmert & Boehmert
Anwaltspartnerschaft mbB
Pettenkoferstrasse 22
80336 München (DE)**

(56) References cited:
JP-A- 2008 029 710    KR-A- 20110 025 921
KR-A- 20150 037 826    US-A1- 2013 273 119
US-B1- 6 503 231

**Description**

TECHNICAL FIELD

The present application claims priority to Korean Patent Application No. 10-2016-0041577 filed on April 5, 2016 in the Republic of Korea.

The present disclosure relates to a soluble microneedle, particularly to a microneedle used to perforate skin which, when inserted through the perforated skin, is dissolved, thereby administering a target material inside the microneedle into the skin.

BACKGROUND ART

Various methods for delivering a target material into the skin with high efficiency have been studied. However, because the skin surface layer has a barrier function of preventing the intrusion of foreign materials into the body, it is very difficult to deliver a target material of an amount sufficient for exerting the desired effect. In addition, it is a very difficult task to provide the target material precisely to a specific site of the skin surface layer. Furthermore, it is particularly difficult to provide a target material which exhibits very low bioavailability or pharmacological availability such as low affinity for skin (lipophilicity), too large molecular weight (500 dalton or higher), etc. through the skin.

Therefore, for sufficient delivery of a target material to a specific site of the skin surface layer regardless of the nature of the target material, the microneedle (microfile, micromissile capsule, etc.) technology has been proposed recently. It is used, for example, for the delivery of cosmetically active substances, drugs, vaccines, etc. into the body, detection of analytes in the body, biopsy, and so forth. The microneedle is made of metals, silicone, etc., self-degradable materials or biodegradable materials.

The microneedle has the characteristic that it can be inserted into the skin without pain or bleeding. However, it is very difficult to make a microneedle which satisfies this characteristic and, at the same time, does not bend or break.

Document KR 2011 0025921 A discloses a solid solution perforator such as a soluble microneedle, wherein: said perforator has a conical shaft; and the outer diameter near the tip of the perforator is 1-100 $\mu$m.

The shape change and perforation rate of the microneedle are principal factors that directly affect the skin permeation rate of the microneedle and the ultimate delivery of the target material. Therefore, there is a consistent need for development of a microneedle in consideration of these factors.

DISCLOSURE

Technical Problem

The present disclosure is directed to providing a microneedle, which exhibits high skin perforation rate and skin permeation rate without having to apply strong force and is capable of effectively delivering a target material into the skin regardless of which part of the microneedle it is contained, and a microneedle patch containing the same.

Technical Solution

According to the present invention there is provided a soluble microneedle, characterized in that the tip diameter of the microneedle being 20 $\mu$m or smaller; or the aspect ratio of the microneedle being 1 -5, wherein the compressive yield strength of the microneedle is 10 kN/cm2 or higher.

Specifically, the present disclosure provides a soluble microneedle having one or more of the following features:

the tip diameter of the microneedle being 10 $\mu$m or smaller; or
the aspect ratio of the microneedle being 1.5-3.

The present disclosure also provides a microneedle patch containing two or more of the microneedle (specifically soluble microneedle) and a support holding the soluble microneedle.

A ratio of the size of the microneedle to the distance between the microneedles may be greater than 0.1 and equal to or smaller than 2, specifically 0.5-2.

Some of the existing microneedles have the problem that, when inserted into the skin by compressing with a hand, the target material is hardly delivered because they are bent. Others are inconvenient to use because they cannot perforate the skin with normal force. If strong pressure is applied to ensure perforation, it leads to breakage of the microneedle or pain. Also, if the microneedle is not sufficiently dissolved in the perforated skin, the target material contained in the microneedle closer to the support of the microneedle remains without being provided to the skin.

The inventors of the present disclosure have studied for a long time and have developed a microneedle and a microneedle patch capable of solving all the problems described above and achieving high skin perforation rate and skin permeation rate by controlling the physical properties of the microneedle, particularly the tip diameter of the microneedle, the aspect ratio of the microneedle and the density of the microneedle in the patch.

In the present disclosure, the skin refers to the surface of a body contacting the external environment. The skin includes the cornea, oral soft tissue, gingiva, nasal mucosa, etc.

In the present disclosure, the application to the skin refers to adhesion, attachment or, specifically, insertion to the skin surface for transdermal administration.

First, the structure of a microneedle according to an exemplary embodiment of the present disclosure is described.

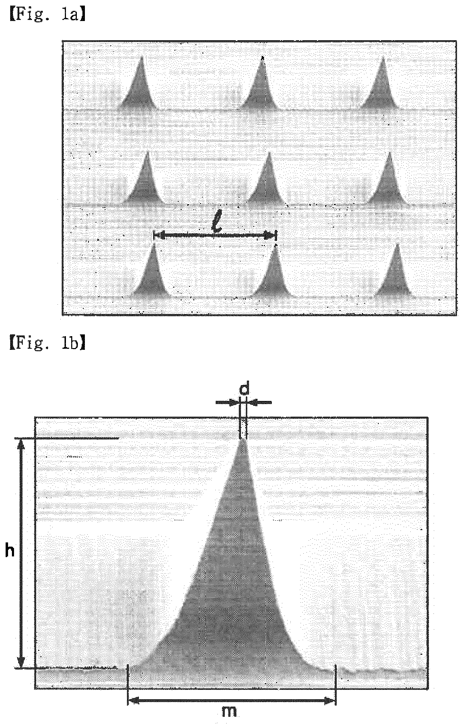

FIGS. 1A and 1B are electron microscopic images of a microneedle fabricated according to an exemplary embodiment of the present disclosure.

FIG. 1A shows a plurality of microneedles formed on the surface of a microneedle support. The microneedles protruding from the microneedle support may form, for example, a microneedle patch.

The microneedles protrude from the microneedle support. Specifically, the microneedle may have a tapered shape. For example, the microneedle is extended away from the support surface as it is tapered from the base of the microneedle toward the tip surface. In the figure, '*l*' means the distance between the microneedles.

FIG. 1B shows the enlarged structure of the microneedle formed on the support surface. In the figure '*h*' means the height of the microneedle. '*m*' means the bottom side of the longitudinal section of the microneedle and '*d*' means the diameter of the microneedle.

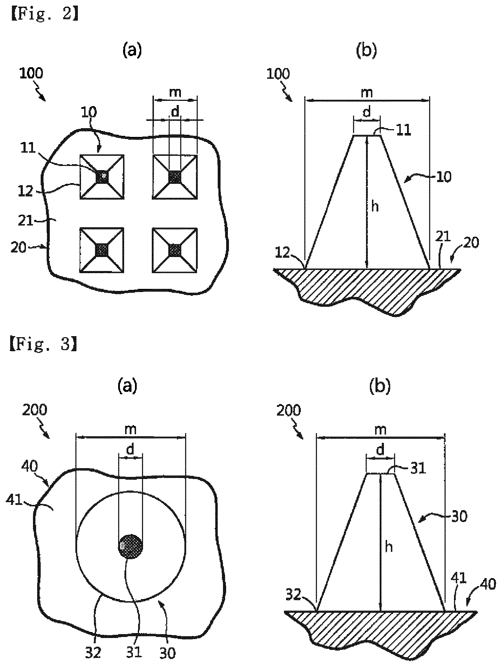

FIG. 2 shows a microneedle 10 with a shape of a tetragonal pyramid having four bottom sides according to an exemplary embodiment of the present disclosure. Although FIG. 2(a) shows a microneedle 10 with a shape of a tetragonal pyramid having four bottom sides, the microneedle may have any appropriate shapes such as a trigonal pyramid, etc. Moreover, the pyramidal microneedle may have a shape of an equilateral pyramid or not. In addition, the microneedle 10 may be a truncated pyramid having a tip surface which may be flat, rather than a perfect pyramid. The truncated pyramid may refer to a pyramid obtained by truncating the sharp tip of the pyramid where all the side faces meet together. The tip surface 11 may be located on a plane which is parallel to the bottom side *m* of the microneedle. Alternatively, the tip surface 11 may be located on a plane which is not parallel to the base 21.

The diameter of the tip surface 11 may be designated as d. In FIG. 2(a), the longest side of the tetragonal tip surface is designated as '*d*' for convenience's sake. However, it may be understood to mean the diameter of a circle having the same area as that of the tip surface.

For the pyramidal (more specifically, truncated pyramidal) microneedle 10, 'the bottom side *m* of the microneedle' may be understood to mean the diameter of a circle having the same area as that occupied by the microneedle base 21 on the support 20.

Although the microneedle 10 shown in FIG. 2(b) has a flat tip surface 11, the tip surface 11 does not need to be perfectly flat. The tip surface 11 may be slightly modified from a completely flat surface but, even in that case, it can be called flat in the context of the present disclosure.

As described above, the microneedle according to the present disclosure is tapered from the wide base adjacent to the support toward the narrow tip surface. FIG. 3 shows that the tapered microneedle 30 is not necessarily restricted to the pyramidal shape.

As seen from FIG. 3, a microneedle patch 200 of the present disclosure may be one wherein one or a plurality of conical microneedle(s) 30 is(are) formed on a microneedle support 40. In another exemplary embodiment of the present disclosure, the microneedle patch may be one wherein a plurality of conical microneedles and a plurality of truncated pyramidal microneedles, which are tapered, are arranged on the support of the microneedle patch.

Referring to FIG. 3, an exemplary embodiment of the present disclosure may provide a conical microneedle 30 formed on a support 40 of a microneedle patch 200.

The area of the conical microneedle 30 occupied by a base 32 may have a circular shape. However, the base 32 may also have different shapes, such as oval, elongated circular or crescent shapes. Although the microneedle is shown to have an equilateral conical shape, the conical microneedle of the present disclosure is not necessarily restricted to the equilateral conical shape. In the conical microneedle, 'the bottom side m of the microneedle' of the present disclosure may be understood to mean the diameter of a circle having the same area as that occupied by the microneedle base 32 on the support 20.

As the truncated pyramid described above, the microneedle according to an exemplary embodiment of the present disclosure is a truncated cone which is truncated at the tip surface 31 which is parallel to the base 32 adjacent to the support surface 41. The tip surface 31 may be located on a plane which is parallel to the base 32 of the microneedle 30. Alternatively, the tip surface 31 may be located on a plane which is not parallel to the base 32.

Although the microneedle 30 shown in FIG. 3 has a flat tip surface 31, the tip surface 31 does not need to be perfectly flat. The tip surface 31 may be slightly modified from a completely flat surface but, even in that case, it can be called flat in the context of the present disclosure.

In the present disclosure, the 'tip' refers to the end of the microneedle which contacts the skin first when the microneedle is applied to the skin. The 'tip surface' refers to the area occupied by the tip when the microneedle is seen from above with the base at the bottom.

When the tip surface has a circular shape, the 'tip diameter' means the diameter of the circle. When the tip surface has a tetragonal shape, it means the diameter of a circle having the same area as that of the tip surface.

In the present disclosure, a 'cross section' refers to the section cut along a horizontal direction. The shape of the cross section may be various depending on the type of the microneedle. For example, the cross section of the microneedle according to an exemplary embodiment of the present disclosure may have a tetragonal or circular shape, although not being limited thereto.

In the microneedle according to the present disclosure, the tip diameter may be 20 $\mu$m or smaller, specifically 19 $\mu$m or smaller, 18 $\mu$m or smaller, 17 $\mu$m or smaller, 16 $\mu$m or smaller, 15 $\mu$m or smaller, 14 $\mu$m or smaller, 13 $\mu$m or smaller, 12 $\mu$m or smaller, 11 $\mu$m or smaller, 10 $\mu$m or smaller, 9 $\mu$m or smaller, 8 $\mu$m or smaller, 7 $\mu$m or smaller, 6 $\mu$m or smaller, 5 $\mu$m or smaller, 4 $\mu$m or smaller, 3 $\mu$m or smaller, 2 $\mu$m or smaller, 1 $\mu$m or smaller, or 0.5 $\mu$m or smaller, most specifically 0.1-10 $\mu$m. The microneedle may more effectively perforate the skin when the tip diameter is 20 $\mu$m or smaller.

In the microneedle according to the present disclosure, an 'aspect ratio' refers to a ratio of the height of the microneedle to its bottom side.

In the present disclosure, the 'height $h$' or 'needle size' refers to the total length of the microneedle, or the linear distance from the tip surface to the base of the microneedle. It can be determined by measuring the height from the longitudinal section of the microneedle. The height of the microneedle according to an exemplary embodiment of the present disclosure may be referred to in FIG. 1B, although not being limited thereto.

In the present disclosure, the 'bottom side $m$' or 'needle base' may be determined by measuring the bottom side length from the longitudinal section of the microneedle.

In the present disclosure, the 'longitudinal section' refers to the section cut along a longitudinal direction. Specifically, the longitudinal section of the microneedle may refer to a section passing through the center of gravity of the microneedle and the center of the tip surface. The longitudinal section of the microneedle according to an exemplary embodiment of the present disclosure may be referred to in FIG. 1B, although not being limited thereto.

In the microneedle according to the present disclosure, the aspect ratio may be 1-5, specifically 1.5-3. If the aspect ratio is smaller than 1, skin perforation may be difficult because the microneedle is blunt. And, if it exceeds 5, skin perforation may be difficult because the microneedle is broken or bent easily.

Also, specifically, the microneedle according to the present invention has compressive yield strength (compressive force / area of microneedle tip) of 10 kN/cm$^2$ or higher. The microneedle is effective for skin perforation when the compressive yield strength is 10 kN/cm$^2$ or higher.

In the present disclosure, the area of the microneedle refers to the area of the microneedle tip surface.

The microneedle according to the present disclosure is not restricted to a specific shape and any shape that is commonly recognized as a microneedle, a microfile, a micromissile capsule, etc. can be included in the scope of the present disclosure. Specifically, it may have a shape which becomes narrower toward the end of the microneedle, e.g., a cone, a polygonal pyramid (trigonal pyramid, quadrangular pyramid, etc.), a combination of a cylinder and a cone, etc., although not being limited thereto. The microneedle according to the present disclosure may have indentation, unevenness, spiral, etc. on its surface.

The microneedle according to the present disclosure may be made of a general synthetic or natural polymer. Specifically, a water-soluble polymer or a self-degradable or biodegradable material may be used.

Specifically, as a material which is dissolved and absorbed in the body when inserted into the skin, hyaluronic acid, sodium carboxymethyl cellulose, a vinylpyrrolidone-vinyl acetate copolymer, polyvinyl alcohol, polyvinylpyrrolidone, a saccharide or a mixture thereof may be used, for example. As the saccharide, xylose, sucrose, maltose, lactose, trehalose or a mixture thereof may be used.

More specifically, hyaluronic acid, sodium carboxymethyl cellulose and a saccharide may be used. Most specifically, a composition for preparing the microneedle may contain 1-60 wt% of sodium carboxymethyl cellulose, 1-60 wt% of hyaluronic acid and 3-60 wt% of a saccharide based on the total weight. Further more specifically, the saccharide may be trehalose. With this composition, the physical properties of the microneedle satisfying the tip diameter and aspect ratio according to the present disclosure and the highest synergistic effect can be achieved.

The microneedle according to the present disclosure may further contain a solubilizer, a plasticizer, a surfactant, a preservative, an anti-inflammatory agent, etc. As the plasticizer, a polyol such as ethylene glycol, propylene glycol, dipropylene glycol, butylene glycol, glycerin, etc. may be used alone or in combination, for example. More specifically, glycerin may be used when considering the result of various evaluations. As the solubilizer, various ingredients known in the art can be adequately selected and used. Specifically, HCO-40 may be used when considering the result of various evaluations of the physical properties and durability of the microneedle and compatibility with other materials forming the microneedle.

The microneedle according to the present disclosure may further contain additional ingredients for achieving synergistic effects depending on the target material.

The microneedle according to the present disclosure may optionally contain various target materials. The target material may be, for example, a drug, a vaccine, a nutrient or a cosmetic ingredient. Also, a cell, a protein, a nucleic acid, a peptide, a polysaccharide, a lipid, etc. may be contained depending on use. Specifically, niacinamide may be used. The target material may be present between the materials forming the microneedle as being dissolved or dispersed in the microneedle or the materials forming the microneedle may enclose the target material, although not being limited thereto.

According to the present disclosure, because the entire microneedle is inserted into and dissolved in the skin, the target material can be administered into the skin regardless of which part of the microneedle it is contained. Even when the target material is contained in a specific zone, even in the rear end of the microneedle, the entire target material may be substantially administered into the skin.

The microneedle according to the present disclosure may be fabricated according to various methods known in the art. For example, it may be fabricated using the materials, fabrication methods, etc. described in Korean Patent Registration No. 10-0793615, International Patent Publication No. WO 02/064193, Korean Patent Publication No. 10-2011-0065361, etc. More specifically, the microneedle according to the present disclosure may be fabricated through solution casting. For example, a mold is prepared first. A liquid composition containing a target material is prepared by dissolving the materials constituting the composition and, optionally, dissolving or dispersing the target material. The liquid composition containing the target material is added to the mold. Specifically, the mold may be subjected to centrifugation or vacuum treatment so that the target material can be spread deep into the liquid composition. Then, the composition is solidified and then taken out from the mold.

The present disclosure also provides a soluble microneedle patch containing two or more soluble microneedles and a support holding the soluble microneedles. The microneedle patch is also called a microneedle device or a microneedle sheet.

According to the present disclosure, the microneedle sheet contains one or more microneedle on at least one side of the support and the microneedle can be inserted into the skin by compressing the microneedle sheet.

In the present disclosure, a ratio of the soluble microneedle size to the distance between the microneedles may be greater than 0.1 and equal to or smaller than 2, more specifically, 0.5-2. If the ratio is 0.1 or smaller, the amount that can be delivered to the skin is limited because the number of microneedles per unit area is too small. And, if it exceeds 2, the microneedle cannot perforate the skin with the force exerted by a user because the microneedles are arranged too densely.

In the present disclosure, the distance between the microneedles refers to the distance between two or more microneedles, which are spaced apart from each other, and can be determined by measuring the linear distance from the tip of one microneedle to the tip of another microneedle.

The description about the size of the microneedle is omitted to avoid redundancy because it is the same as defined above.

In the present disclosure, the support may be made of the same material as the microneedle and may additionally contain a material having adhesion affinity. The material having adhesion affinity may include, for example, an acryl-based or rubber-based adhesive such as polyurethane, polyethylene, polyester, polypropylene, polyvinyl chloride, etc. or a hydrocolloid wherein hydrophilic hydrocolloid particles are dispersed in a hydrophobic polymer matrix, etc., although not being limited thereto.

Advantageous Effects

Because a microneedle according to the present disclosure has appropriate mechanical strength, viscosity and hardness, it can be easily inserted into the skin without change in shape such as bending or breaking and enables efficient skin perforation. In addition, because the microneedle according to the present disclosure is dissolved in the perforated skin and exhibits superior skin permeation rate of the material, there is no need of applying strong force and the target material can be effectively delivered into the skin regardless of which part of the microneedle it is contained.

DESCRIPTION OF DRAWINGS

The accompanying drawings illustrate a preferred embodiment of the present disclosure and together with the foregoing disclosure, serve to provide further understanding of the technical features of the present disclosure, and thus, the present disclosure is not construed as being limited to the drawings.

FIG. 1A is a front image of a microneedle according to an exemplary embodiment of the present disclosure.

FIG. IB is an enlarged image of one microneedle according to an exemplary embodiment of the present disclosure, illustrating the measurement of the bottom side m and height h of the microneedle.

FIG. 2 shows a microneedle 10 according to an exemplary embodiment of the present disclosure in the form of a truncated pyramid having four sides. In FIG. 2, the tip surface has a tetragonal shape. (a) is a view of the tip surface 11 seen from above and (b) shows the longitudinal section of the microneedle 10 protruding from a support 20.

FIG. 3 shows a microneedle 30 according to an exemplary embodiment of the present disclosure 30 in the form of a truncated cone. In FIG. 3, the tip surface has a circular shape. (a) is a view of the tip surface 31 seen from above and (b) shows the longitudinal section of the microneedle 30 protruding from a support 40.

FIG. 4 shows the result of microneedle skin perforation tests in Test Example 1, Comparative Example 1, Comparative Example 2-1 and Comparative Example 3-2.

FIG. 5 shows the result of compressive yield strength test of a microneedle in Test Example 1. The x-axis indicates compressed length and the y-axis indicates compressive force.

FIG. 6 shows a Franz diffusion cell designed to evaluate the release behavior of a drug contained in a microneedle.

FIG. 7 shows the skin permeation rate of a microneedle depending on time as the result of microneedle skin perforation tests in Test Example 1, Comparative Example 1, Test Example 2-1 and Test Example 3-1.

FIG. 8 shows electron microscopic images showing the result of microneedle solubility tests in Test Example 1, Comparative Example 1 and Comparative Example 2-2.

m: bottom side of microneedle
h: height of microneedle
d: tip diameter
$\ell$: microneedle distance
100, 200: microneedle patch
10, 30: microneedle
11, 31: tip surface
12, 32: base
20, 40: support
21, 41: support surface

BEST MODE

Hereinafter, preferred embodiments of the present disclosure will be described in detail with reference to the accompanying drawings. Prior to the description, it should be understood that the terms used in the specification and the appended claims should not be construed as limited to general and dictionary meanings, but interpreted based on the meanings and concepts corresponding to technical aspects of the present disclosure on the basis of the principle that the inventor is allowed to define terms appropriately for the best explanation. Therefore, the description proposed herein is just a preferable example for the purpose of illustrations only, not intended to limit the scope of the disclosure, so it should be understood that other equivalents and modifications can be made thereto without departing from the scope of the disclosure.

Fabrication of soluble microneedle

A microneedle was fabricated as described in Table 1. In the present disclosure, the contents are in wt% units unless specified otherwise.

A solution was prepared by dissolving HA (hyaluronic acid), Na-CMC (sodium carboxymethyl cellulose) and trehalose in purified water and then adding glycerin, HCO-40 and niacinamide. The prepared solution was cast in a silicone microneedle mold and then centrifuged at 3000 rpm for 10 minutes. The resulting solution was filled in a microneedle mold.

After drying in a drying oven (70 °C) for 3 hours, the fabricated microneedle was separated from the microneedle mold using an adhesive film.

[Table 1]

| Fabrication Example | |
|---|---|
| HA | 5 |
| Na-CMC | 10 |
| Trehalose | 10 |

(continued)

| Fabrication Example | |
|---|---|
| Glycerin | 5 |
| HCO-40 | 0.2 |
| Niacinamide | 5 |
| Water | To 100 |

Microneedle structure

Tip diameter, aspect ratio and needle density were controlled as described in Table 2. The values were measured from SEM (electron microscopic) images. As the HA, low-molecular-weight HA having a molecular weight of about 30,000 dalton was used.

[Table 2]

| | Tip diameter | Aspect ratio | Needle density | Remarks |
|---|---|---|---|---|
| Test Example 1 | 5 $\mu$m | 2.5 | 0.5 | Normal |
| Test Example 2 | 10 $\mu$m | 2.5 | 0.5 | Normal |
| Test Example 3 | 15 $\mu$m | 2.5 | 0.5 | Normal |
| Test Example 4 | 5 $\mu$m | 2.0 | 0.5 | Normal |
| Test Example 5 | 5 $\mu$m | 1.5 | 0.5 | Normal |
| Comparative Example 1 | 30 $\mu$m | 2.5 | 0.5 | Tip diameter |
| Comparative Example 2-1 | 5 $\mu$m | 0.5 | 0.5 | Aspect ratio |
| Comparative Example 2-2 | 5 $\mu$m | 5 | 0.5 | |
| Comparative Example 3-1 | 5 $\mu$m | 2.5 | 0.1 | Needle density |
| Comparative Example 3-2 | 5 $\mu$m | 2.5 | 2 | |

$$\text{Aspect ratio} = \text{height of microneedle} / \text{bottom side of microneedle}$$

$$\text{Needle density} = \text{microneedle size} / \text{distance between microneedles}$$

Skin perforation test of the soluble microneedle

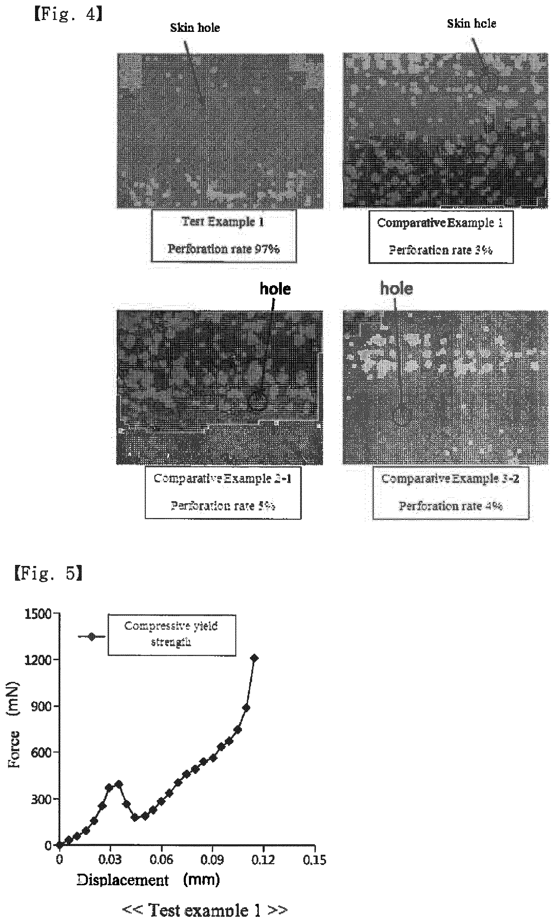

The soluble microneedle was attached to pig skin and the skin was perforated by compressing for 10 seconds with 30 N/cm$^2$ (exerted by a user). Then, the microneedle was removed immediately. The pig skin was stained with a trypan blue solution and the solution adhering to the surface was wiped with a swab. Then, the hole formed by the microneedle was observed with an optical microscope.
The result is shown in FIG. 4.

$$\text{Skin perforation rate (\%)} = (\text{number of skin holes} / \text{number of microneedles}) \times 100$$

Test Examples 1-5 showed high skin perforation rates, whereas Comparative Example 1, Comparative Example 2-1 and Comparative Example 3-2 showed very low skin perforation rates of 3%, 5% and 4%, respectively.

Compressive yield strength test of the soluble microneedle

The compressive yield strength of the soluble microneedle was measured using a texture analyzer. Test Example 1 showed a compressive yield strength of 1,500 kN/cm² (FIG. 5).

$$\text{Compressive yield strength (kN/cm}^2) = \text{compressive force / area of microneedle tip}$$

When the compressive yield strength was 10 kN/cm² orl ower, the skin could not be perforated because the microneedle was bent.

Drug release behavior test

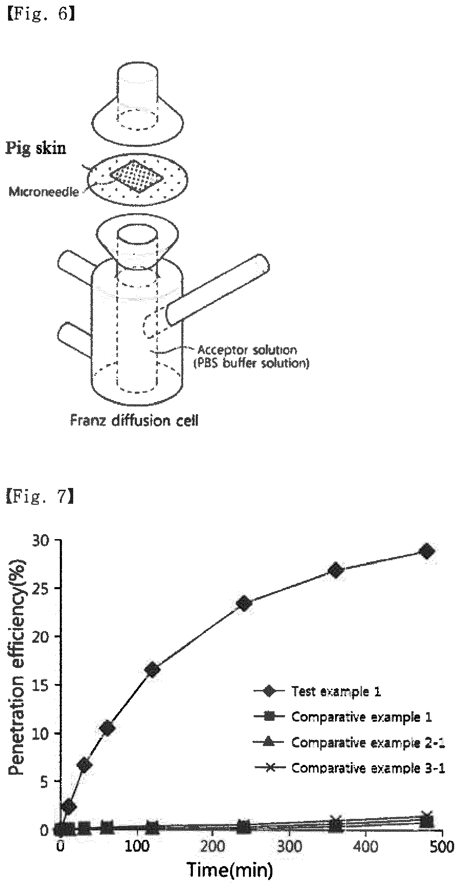

A drug release behavior was conducted using a Franz diffusion cell (FIG. 6) by measuring the content of a drug (niacinamide) in an acceptor solution with time by liquid chromatography. A more detailed procedure is as follows.
After attaching a niacinamide-filled microneedle patch to pig skin, the skin permeation rate of niacinamide with time was compared.

$$\text{Skin permeation rate (\%)} = \text{(amount permeated into skin / amount filled in}$$

$$\text{microneedle)} \times 100$$

The amount permeated into the skin corresponds to the sum of the drug content in the pig skin tissue and the drug content in the acceptor solution.
As seen from FIG. 7, Test Example 1 showed fast increase in the skin permeation rate with time, whereas Comparative Example 1, Comparative Example 2-1 and Comparative Example 3-1 showed little increase of the skin permeation rate of niacinamide. Accordingly, it can be seen that the microneedle according to the test example is more effective in permeating the drug into the skin as compared to the comparative examples.
Skin perforation rate and skin permeation rate of microneedle

[Table 3]

| | Tip diameter | Aspect ratio | Needle density | Skin perforation rate (%) | Skin permeation rate (%) |
|---|---|---|---|---|---|
| Test Example 1 | 5 μm | 2.5 | 0.5 | 97% | 28.7% |
| Test Example 2 | 10 μm | 2.5 | 0.5 | 90% | 20.5% |
| Test Example 3 | 15 μm | 2.5 | 0.5 | 75% | 15.5% |
| Test Example 4 | 5 μm | 2.0 | 0.5 | 92% | 28.2% |
| Test Example 5 | 5 μm | 1.5 | 0.5 | 82% | 26.7% |
| Comparative Example 1 | 30 μm | 2.5 | 0.5 | 3% | 0.7% |
| Comparative Example 2-1 | 5 μm | 0.5 | 0.5 | 5% | 1.0% |
| Comparative Example 2-2 | 5 μm | 5 | 0.5 | 4% | 0.7% |
| Comparative Example 3-1 | 5 μm | 2.5 | 0.1 | 98% | 1.5% |
| Comparative Example 3-2 | 5 μm | 2.5 | 2 | 4% | 0.8% |

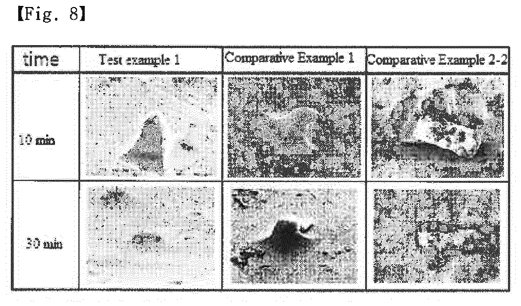

The tip diameter, aspect ratio, microneedle density, skin perforation rate, skin permeation rate of the test examples and comparative examples are shown in Table 3.
Test Examples 1-5 showed high skin perforation rate and, therefore, high skin permeation rate of the drug.
In contrast, Comparative Example 1, wherein the tip diameter was 30 μm, showed very low skin perforation rate. As can be seen from FIG. 8, the skin permeation rate was very low because the microneedle was bent when compressed.

For Comparative Example 2-1, wherein the aspect ratio was 0.5, the skin perforation rate was very low because the microneedle was blunt. For Comparative Example 2-2, wherein the aspect ratio was 5, the microneedle was bent when compressed as can be seen from FIG. 8 and, accordingly, showed very low skin perforation rate.

Comparative Example 3-1 showed high skin perforation rate, but the skin permeation rate of the drug was low due to low microneedle density. For Comparative Example 3-2, the microneedle could not easily perforate the skin with the force exerted by a user because the microneedles were arranged too densely and, accordingly, the skin permeation rate was low.

INDUSTRIAL APPLICABILITY

The present disclosure may provide a microneedle that can be dissolved in skin and a patch product containing the microneedle. Because the microneedle has appropriate mechanical strength, viscosity and hardness, it can be easily inserted into the skin without change in shape such as bending or breaking and enables efficient skin perforation.

**Claims**

1. A soluble microneedle (10), **characterized in that** the tip diameter of the microneedle (10) being 20 $\mu$m or smaller; or the aspect ratio of the microneedle (10) being 1-5,
wherein the compressive yield strength of the microneedle (10) is 10 kN/cm$^2$ or higher.

2. The soluble microneedle (10) according to claim 1, wherein
the tip diameter of the microneedle (10) being 10 $\mu$m or smaller; or
the aspect ratio of the microneedle (10) being 1.5-3.

3. The soluble microneedle (10) according to claim 1, wherein
the tip diameter of the microneedle (10) being 10 $\mu$m or smaller; and
the aspect ratio of the microneedle (10) being 1.5-3.

4. The soluble microneedle (10) according to claim 1, wherein the microneedle (10) comprises hyaluronic acid, sodium carboxymethyl cellulose and a saccharide.

5. A microneedle patch (200) comprising two or more of the soluble microneedle (10) according to any of claims 1 to 3 and a support (20) holding the soluble microneedle (10).

6. The microneedle patch (200) according to claim 5, wherein a ratio of the size of the microneedle (10) to the distance between the microneedles (10) is greater than 0.1 and equal to or smaller than 2.

7. The microneedle patch (200) according to claim 6, wherein the ratio of the size of the microneedle (10) to the distance between the microneedles (10) is 0.5-2.

**Patentansprüche**

1. Lösliche Mikronadel (10), **dadurch gekennzeichnet, dass** der Durchmesser der Spitze der Mikronadel (10) 20 $\mu$m oder kleiner ist oder das Seitenverhältnis der Mikronadel (10) 1 bis 5 beträgt,
wobei die Druckfestigkeit der Mikronadel (10) 10 kN/cm$^2$ oder höher ist.

2. Lösliche Mikronadel (10) nach Anspruch 1, bei der der Durchmesser der Spitze der Mikronadel (10) 10 $\mu$m oder kleiner ist oder das Seitenverhältnis der Mikronadel (10) 1,5 bis 3 beträgt.

3. Lösliche Mikronadel (10) nach Anspruch 1, bei der der Durchmesser der Spitze der Mikronadel (10) 10 $\mu$m oder kleiner ist und das Seitenverhältnis der Mikronadel (10) beträgt 1,5-3.

4. Lösliche Mikronadel (10) nach Anspruch 1, bei der die Mikronadel (10) Hyaluronsäure, Natrium-Carboxymethylcellulose und ein Saccharid umfasst.

5. Mikronadelpflaster (200), das zwei oder mehrere der löslichen Mikronadeln (10) nach einem der Ansprüche 1 bis 3 und einen Träger (20) umfasst, der die lösliche Mikronadel (10) hält.

6. Mikronadelpflaster (200) nach Anspruch 5, bei dem das Verhältnis der Größe der Mikronadel (10) zum Abstand zwischen den Mikronadeln (10) größer als 0,1 und gleich oder kleiner als 2 ist.

7. Mikronadelpflaster (200) nach Anspruch 6, bei dem das Verhältnis der Größe der Mikronadel (10) zu dem Abstand zwischen den Mikronadeln (10) 0,5 bis 2 beträgt.

**Revendications**

1. Micro-aiguille soluble (10), **caractérisée en ce que** le diamètre de la pointe de la micro-aiguille (10) est de 20 $\mu$m ou plus petit ; ou bien
le rapport de longueur sur largeur de la micro-aiguille (10) est de 1-5,
où la limite d'élasticité en compression de la micro-aiguille (10) est de 10 kN/cm$^2$ ou plus élevée.

2. Micro-aiguille soluble (10) selon la revendication 1, où le diamètre de la pointe de la micro-aiguille (10) est de 10 $\mu$m ou plus petit ; ou bien
le rapport de longueur sur largeur de la micro-aiguille (10) est de 1,5-3.

3. Micro-aiguille soluble (10) selon la revendication 1, où le diamètre de la pointe de la micro-aiguille (10) est de 10 $\mu$m ou plus petit ; ou bien
le rapport de longueur sur largeur de la micro-aiguille (10) est de 1,5-3.

4. Micro-aiguille (10) selon la revendication 1, où la micro-aiguille (10) comprend de l'acide hyaluronique, de la carboxyméthylcellulose de sodium et un saccharide.

5. Patch de micro-aiguilles (200) comprenant deux ou plus de la micro-aiguille soluble (10) selon l'une quelconque des revendications 1 à 3 et un support (20) tenant la micro-aiguille soluble (10).

6. Patch de micro-aiguilles (200) selon la revendication 5, où un rapport de la taille de la micro-aiguille (10) à la distance entre les micro-aiguilles (10) est supérieur à 0,1 et égal ou inférieur à 2.

7. Patch de micro-aiguilles (200) selon la revendication 6, où le rapport de la taille de la micro-aiguille (10) à la distance entre les micro-aiguilles (10) est de 0,5-2.

【Fig. 1a】

【Fig. 1b】

[Fig. 2]

(a)

(b)

[Fig. 3]

(a)

(b)

【Fig. 4】

Skin hole

Skin hole

Test Example 1

Perforation rate 97%

Comparative Example 1

Perforation rate 3%

hole

hole

Comparative Example 2-1

Perforation rate 5%

Comparative Example 3-2

Perforation rate 4%

【Fig. 5】

<< Test example 1 >>

【Fig. 6】

【Fig. 7】

【Fig. 8】

| time | Test example 1 | Comparative Example 1 | Comparative Example 2-2 |
|---|---|---|---|
| 10 min | | | |
| 30 min | | | |

**EP 3 441 106 B1**

## REFERENCES CITED IN THE DESCRIPTION

**Patent documents cited in the description**

- KR 1020160041577
- KR 20110025921 A
- KR 100793615
- WO 02064193 A
- KR 1020110065361